# EUROPÄISCHE PATENTSCHRIFT

(11) **EP 1 450 825 B1**
(45) Veröffentlichungstag und Bekanntmachung des Hinweises auf die Patenterteilung: **13.08.2008**
(21) Anmeldenummer: 02792569.2
(22) Anmeldetag: 04.12.2002
(51) Int. Cl.: A61K 33/04, A61P 29/00, A61P 1/02, A61P 17/00, A61P 31/12

(54) **VERWENDUNG VON SELENITHÄLTIGEN VERBINDUNGEN ZUR TOPISCHEN ODER BUKKALEN ANWENDUNG**
USE OF SELENITE-CONTAINING COMPOUNDS TO BE TOPICALLY OR BUCCALLY ADMINISTERED
UTILISATION DE COMPOSES CONTENANT DU SELENITE POUR PRODUIRE UN AGENT A ADMINISTRER PAR VOIE TOPIQUE OU BUCCALE

(30) Priorität: 04.12.2001 AT 18952001
(43) Veröffentlichungstag der Anmeldung: 01.09.2004
(73) Patentinhaber: vis-vitalis Lizenz- und Handels GmbH, 5081 Anif (AT)
(72) Erfinder: KUKLINSKI, Bodo, 18055 Rostock (DE); KÖSSLER, Peter, A-5571 Mariapfarr (AT); FUCHS, Norbert, A-5571 Mariapfarr (AT)
(74) Vertreter: Sonn & Partner Patentanwälte
(86) Internationale Anmeldenummer: PCT/AT2002/000336
(87) Internationale Veröffentlichungsnummer: WO 2003/047604

(56) Entgegenhaltungen:
- EP-A- 0 000 670
- EP-A- 0 750 911
- WO-A-00/12101
- WO-A-01/93910
- WO-A-02/072112
- DE-A- 4 320 694
- FR-A- 2 779 720
- US-A- 4 512 977
- US-A- 4 668 515

## Beschreibung

Die Erfindung betrifft neue Verwendungen selenhältiger wässriger Lösungen sowie pharmazeutisch verabreichbarer oder lebensmittelkompatibler Selenpräparate.

Sämtliche Stoffwechselprozesse in organischen Lebewesen (Pflanze, Tier, Mensch) im Sinne von Wachstums-, Differenzierungs- und Energie-Prozessen sind auf biochemischer Ebene ein Wechselspiel von reduktiven und oxidativen Prozessen. Diese "Redox-Vorgänge" sind letztlich Ausdruck der Elektronen-Übertragung von biochemischen Reduktions-Äquivälenten, wie z.B. NADH + H⁺ (Elektronendonator) auf atmosphärischen, molekularen Sauerstoff als Oxidationsmittel (Elektronen-Akzeptor). Die Oxidation unserer Nährstoffe (Fette; Kohlenhydrate, Proteine, Sauerstoff) dient der permanenten Erhaltung und Entwicklung unserer biologischen Strukturen.

Andererseits aber bestehen gerade unsere zellulären und subzellulären Strukturen, die daraus gebildeten Gewebe und Organe und letztlich jedes organische Individuum in ihrer Gesamtheit aus gerade jenen Strukturen (Nährstoffen), die zur Erhaltung und Entwicklung lebender Organismen ständig von außen zugeführt, zu Energiegewinnung oxidiert, sogleich aber auch zur Erhaltung der funktionellen, anatomischen und histologischen Struktur dienen müssen. Somit sind diese biologischen Strukturen letztlich ebenso oxidabel wie jene Nährstoffe, die zur Aufrechterhaltung unserer Lebensenergie oxidiert werden müssen. Um eine "Autooxidation" biologischer Strukturen zu verhindern, bedient sich der organische lebende Organismus endogener und exogener "Antioxidantien". Zu den endogenen Antioxidantien gehören unter anderem Enzyme und Enzym-Systeme, wie die Superoxiddismutase, Katalasen, Peroxydasen, Cholesterin und reduziertes Glutathion, während exogene Antioxidantien, z.B. Vitamin A, β-Carotin, Vitamin E, Vitamin C oder Selen darstellen.

Das Maß für die "antioxidative Kapazität", also die Bereitschaft, Elektronen auf andere Atome und Moleküle zu übertragen, wird im sogenannten "Reduktionspotential" (Standard-Redoxpotential) quantitativ ausgedrückt. Nachfolgende Tabelle 1 gibt einen Überblick über die Standard-Redoxpotentiale einiger endogener und exogener Antioxidantien organischen Lebewesen:

**Tabelle 1**

| **Standard-Redoxpotentiale einiger Antioxidantien** | |
|---|---|
| **Eₒ (Volt)** | **System** |
| + 0,82 | O₂/H₂O |
| + 0,366 (basisches Milieu) | Selenit |
| + 0,300 | Tocopherol (Vitamin E) |
| + 0,100 | Ubiquinon (Coenzym Q₁₀) |
| + 0,08 | Ascorbinsäure |
| + 0 (+0,16 bis -0,02 V) | Flavonoide |
| - 0,12 | Riboflavin (Vitamin B₂) |
| - 0,22 | Cystin/Cystein |
| - 0,23 | G SH/GSSG |
| - 0,29 | Thioctsäure (α-Liponsäure) |
| - 0,32 | NADH + H⁺/NAD |
| - 0,740 (saures Milieu) | Selenit |

Antioxidantien sind somit Atome und Moleküle (für den humanen Organismus, vor allem Nährstoffmoleküle und Enzym-Komplexe), die schneller mit Stoffwechselradikalen reagieren als biologische Strukturen. Sie schützen somit unsere Zell-, Gen- und Bindegewebs-Strukturen dadurch, dass sie Stoffwechselzündfunken (Radikale, Peroxide) abfangen, bevor diese z.B. ungesättigte Fettsäuren unserer Biomembranen oder schwefelhaltige Bauteile lebenswichtiger Struktur- oder Enzymproteine angreifen. Wie oben stehende Tabelle zeigt, verändern bestimmte Elemente, wie z.B. Selen, ihr Standard-Redopotential durch Veränderung des pH-Milieus, in dem diese Verbindungen gelöst sind.

Selen ist ein essentielles Spurenelement für höhere Tiere und den Menschen. Es besitzt eine Schutzfunktion für Proteine gegen Oxidation, die z.B. durch die Glutathion-Peroxidase erfolgt, die die Aminosäure Selenocystein im aktiven Zentrum enthält. Selenmangel wird mit Rheumatismus und Grauem Star in Verbindung gebracht, die Keshan-Krankheit, die in einigen Gebieten Chinas verbreitet ist, gilt als Selen-Mangelerscheinung. Selenite können die Wirkung von Vitamin E steigern und sorgen für die Entgiftung von Quecksilber und Cadmium. Eine Schutzwirkung von Selen vor Karzinogenen wird ebenfalls postuliert.

Andererseits wirkt Selen in höheren Konzentrationen toxisch, wobei die Toxizität darauf zurückgeführt wird, dass Selen den Schwefel in Proteinen verdrängen kann. Die Ausscheidung erfolgt in der Regel als Selenat über die Niere und den Darm. Erkrankungen des menschlichen Körpers werden hervorgerufen, wenn die tägliche Nahrung mehr als 1 µg Selen/g enthält (wobei ein Mindestgehalt von 0,02 µg Selen/g erforderlich ist, um Mangelerscheinungen vorzubeugen). Insgesamt enthält der menschliche Körper ca. 10 bis 15 µg Selen.

Auch bei Tieren treten Vergiftungserscheinungen bei mehr als 5 bis 10 µg Selen/g in der Tiernahrung auf, beispielsweise Hemmung des Wachstums, Haarausfall, Erweichung der Hörner und Hufe, bei Vögel Federausfall. Jedoch ist auch bei Tieren Selen zur Aufzucht von Küken, Puten und Schweinen notwendig, ebenso wie zur Vermeidung spezifischer Erkrankungen bei Nutztieren, insbesondere Schafen. Daher ist Natriumselenit und Natriumselenat als Mischfutterzusatz oder zur Düngung von Weiden erforderlich, da der natürlicher Selengehalt tierischer und pflanzlicher Futtermittel oft unzureichend ist oder das Element nicht ausreichend freigesetzt wird.

In der US 4 668 515 wird ein selenhältiges Getränk, welches mit Zitronensäure und Ascorbinsäure gemischt wird, beschrieben, wobei die Lösung einen pH von 2,75 oder höher aufweisen soll. Gemäß diesem Dokument geht es vor allem darum, durch Zusatz von Zitronen- und Ascorbinsäure auf einen pH von mehr als 2,75 zu gelangen, da - so die Prämisse dieses Dokuments - ansonsten Natriumselenit durch Säuren inaktiviert wird. Hierbei werden also Säuren zugesetzt, um pH-Stabilität zu gewährleisten.

Ein Effekt auf das Standard-Redox-Potential tritt bereits beim Einsatz von nur einer ausgewählten Säure auf, wird jedoch hier nicht erwähnt.

Weiters wird in der US 4 668 515 lediglich die orale Verabreichung dieser Präparate zur Beibehaltung der Gesundheit sowie zur Verhinderung von der Bildung von spontanen Brustdrüsentumoren beschrieben.

In der DE 44 37 403 A1 werden Antioxidativa beschrieben, die unter anderem Selen in organischer Bindung enthalten. Der Gegenstand dieses Dokuments beruht auf der Kombination von verschiedenen antioxidativen Substanzen, welche angeblich in einem deutlichen synergistischen Effekt resultiert.

Die Antioxidantienkombination gemäß der DE 44 37 403 A1 ist jedoch ebenfalls nur zur inneren Anwendung vorgesehen (wobei die in dieser Entgegenhaltung beschriebene Mischung eine äußerst komplexe Vitamin- und Spurenelementemischung darstellt).

Schließlich betrifft die DE 43 35 441 A1 Mittel zur Vorbeugung von Herz-/Kreislauf-Erkrankungen, enthaltend Salicylsäure-Derivate sowie eine selenhältige Verbindung. Acetylsalicylsäure ist bekanntlich ein Pharmakon zur Verbesserung der Fließeigenschaften des Blutes.

Die EP 0 000 670 A1 offenbart pharmazeutische Formulierungen, die Selenite oder Selenate enthalten können, jedoch auch Ascorbinsäure. Die Kombination von Selenit (bzw. dessen jeweils vorhandener Oxidationsstufe) und Ascorbinsäure führt zu unerwünschten Redoxreaktionen (Selenit wird von Ascorbinsäure zu Selen reduziert), die das erhöhte anti-oxidative Potential einer Kombination von Selenit mit Säuren reduzieren.

In der US 5 648 389 werden dermatologische Störungen erwähnt. Diese Störungen werden durch ein Mittel behandelt, welches Glykolsäure, Salicylsäure oder Milchsäure, sowie eine dermatologische absorbierbare Zinkverbindung, z.B. Zinkselenat, als wesentliche Wirkkomponente enthält.

Die CN 1 126 042 A betrifft gemäß der Zusammenfassung eine Creme zur Gesundheitspflege, die reich an γ-Natrium-Linolenat (bzw. Linolensäure (gemäß dem Titel)) und Selen ist, welche Creme aus Pflanzenöl und Natriumselenit besteht.

Die DE 43 20 694 C2 offenbart die Verwendung von Selenverbindungen zur äußeren Anwendung bei Warzen. Eine Kombination mit Säuren wird aber hierbei weder beschrieben noch nahe gelegt.

In der DE 44 19 783 A1 wird ein Haarkur-Pflege-Shampoo beschrieben, welches organische Säuren und unter anderem Selensalze enthält.

Die US 4 512 977 betrifft Selen-hältige Verbindungen zur Behandlung von Verletzungen.

In der WO 00/12101 A werden Selen-hältige Zusammensetzungen zur Kontrolle von oxidativem Stress und exzessiven entzündlichen Reaktionen beschrieben.

Eine organische Selenverbindung zur Behandlung von Hauterkrankungen geht aus der FR 2 779 720 A hervor.

Die EP 0 750 911 A betrifft Selen-hältige Verbindungen zur Verhinderung oder Behandlung von Krebs oder viralen Erkrankungen.

Aufgabe der vorliegenden Erfindung war es, neue Verwendungen für selenhältige Präparate zur Verfügung zu stellen und selenit- oder selenathältige Präparate neuen Lebensmittel-/Futtermitteltechnologischen sowie pharmazeutischen Verwendungen zuzuführen bzw. hinsichtlich ihrer Wirkung in diesen Gebieten zu verbessern.

Diese Aufgabe wird erfindungsgemäß gelöst durch die Verwendung von selenithältigen Verbindungen und pharmazeutisch akzeptablen Säuren, ausgewählt aus Zitronensäure, Essigsäure, Apfelsäure, Kohlensäure, Schwefelsäure, Salpetersäure, Salzsäure, diverse Fruchtsäuren oder Mischungen davon, zur Herstellung eines Mittels zur topischen oder bukkalen Anwendung bzw. zur mukosalen Verabreichung bei der Behandlung von Infektionen mit Papillomaviren.

Es hat sich gezeigt, dass durch Versetzen von wässerigen Lösungen anorganischer Selen-Verbindungen mit derartigen Säuren eine Zusammensetzung mit erhöhtem antioxidativen Potential zur Verfügung gestellt werden kann. Die erfindungsgemäß hergestellten Zusammensetzungen, also insbesondere Lösungen, Gele, Emulsionen, Suspensionen, Salben usw., zeigen dabei auch überraschende therapeutische Effekte, wenn sie in einer Weise angewendet werden, in der dieses erhöhte antioxidative Potential zumindest zeitweise erhalten bleibt. Dies ist dann der Fall, wenn bei der Anwendung am therapeutischen Zielort das gesteigerte antioxidative Potential noch vorhanden ist und nicht beispielsweise durch Applikationslösungen oder Körpersäfte wie Blut (z.B. bei intravenöser Anwendung) oder Magen- oder Darminhalte (bei oraler Anwendung) verdünnt worden ist. Demgemäß betrifft die vorliegende Erfindung die Verwendung dieser Präparate für die externe Anwendung (also topisch oder bukkal) bzw. für die direkte Applikation an Schleimhäute (mukosale Anwendung).

Es zeigte sich, dass sich die erfindungsgemäße Verwendung für eine breite Reihe an Krankheitsbildern, insbesondere prooxidativen, eignet, wobei in vielen Fällen die erfindungsgemäße Behandlung bzw. das erfindungsgemäß zu verwendete Mittel mit weiteren Therapiemaßnahmen kombiniert werden kann.

Vorzugsweise wird dem erfindungsgemäß zu verwendende Mittel ein pharmazeutisch akzeptabler Träger, vorzugsweise Siliziumdioxid, insbesondere hochdisperses Siliziumdioxid, zugesetzt, der speziell für die Art der Applikation hergerichtet ist und sich dann ganz speziell zu topischer bzw. bukkaler Verabreichung oder zur Verabreichung an eine Mukosa eignet. Derartige anwendungsspezifische Zusatzstoffe bzw. pharmazeutisch akzeptable Träger sind dem Fachmann auf dem vorliegenden Gebiet für die jeweils gewünschte Anwendungsform bzw. sogar für die jeweilige spezifische Mukosa ausreichend bekannt und ohne weiteres auf den Gegenstand der vorliegenden Erfindung übertragbar.

Bevorzugterweise weist das erfindungsgemäß zu verwendende selenithältige Mittel bei der Anwendung einen pH von unter 7,0, vorzugsweise unter 5,0, insbesondere unter 4,0, auf. Besonders bevorzugte erfindungsgemäße Mittel weisen dabei einen pH von 6,0 bis 2,0, insbesondere 3,0 bis 2,5, auf.

An sich ist die Natur der zugesetzten lebensmittelkompatiblen oder pharmazeutisch verabreichbaren Säure nicht kritisch. Bei Ascorbinsäure kann es jedoch zur Reduktion der anorganischen Selenverbindung zu elementarem Selen kommen, die in vielen Fällen für Fertigpräparate ungeeignet ist. Bevorzugterweise werden demgemäß Säuren verwendet, bei welchen eine Reduktion von anorganischen Selenverbindungen zu elementarem Selen nicht eintritt. Insbesondere Zitronensäure, Essigsäure, Apfelsäure, Kohlensäure, sowie andere organische und anorganische Säuren, wie z.B. Schwefelsäure, Salpetersäure, Salzsäure, diverse Fruchtsäuren oder Mischungen dieser Säuren, sind also für die vorliegende Erfindung besonders gut geeignet.

Die erfindungsgemäß zu verwendende Zusammensetzung kann nicht nur in wässeriger Lösung vorgesehen werden. Bevorzugte weitere Formen stellen die Salbenform, die Gelform oder die Emulsion dar, die sich insbesondere für die erfindungsgemäße topische, bukkale oder mukosale Verabreichung eignen.

Selbstverständlich kann das erfindungsgemäß zu verwendende Mittel weiters Hilfsstoffe, wie Puffersubstanzen, Farbstoffe, Stabilisierungsmittel oder Trägersubstanzen und/oder weitere wirksame Komponenten, wie z.B. Antibiotika, antivirale Mittel, Antimykotika, schmerzhemmende Mittel oder entzündungshemmende Mittel enthalten, wobei diese Hilfsstoffe auch in allen möglichen Kombinationen verwendet werden können. Die jeweilige Art des Hilfsstoffes bzw. der weiteren wirksamen Komponente richtet sich nach der jeweiligen Verwendung im Einzelfall. Bevorzugterweise ist das Mittel auf Trägermaterialien, vorzugsweise medizinische Schwämme und/oder andere adsorbierende Materialien, insbesondere Wundkegel, aufgetragen.

Bei nässenden Erkrankungen erweist sich die Zugabe physikalisch adsorbierender Hilfsstoffe vom Typ der hochdispersen Silikate als besonders vorteilhaft.

Die Erfindung wird an Hand der nachfolgenden Beispiele, auf die sie jedoch selbstverständlich nicht eingeschränkt ist, näher erläutert.

### Beispiele :

### Beispiel 1 : Herstellung einer angesäuerten Natriumselenit-Lösung:

Eine angesäuerte Natriumselenit-Lösung wurde mit folgender Zusammensetzung hergestellt (pro 100 ml):

| | |
|---|---|
| Natriumselenit-Pentahydrat | 0,111 g |
| Maltodextrin | 0,5 g |
| Zitronenaroma | 0,1 g |
| Zitronensäure | 0,5 g |
| Lebensmittelfarbe | 0,01 g |
| Kaliumsorbat | 0,1 g |
| Natrium-Benzoat | 0,05 g |
| Aqua destillata | 99,29 g |

### Beispiel 2 : Behandlung von Papillomatosen

Papillomatosen sind Blumenkohl-artige Wucherungen, die durch das sogenannte Papilloma-Virus hervorgerufen werden. Papillome sind aus histologischer Sicht meist benigne Öberflächenepithel-Tumore, lokalisiert auf der Mundschleimhaut, den ableitenden Harnwegen, an den Ober- und Unterschenkeln sowie an der Anal- und Genital-Schleimhaut. Eine Progression durch Papilloma-Viren-induzierte benigne Tumore zu Karzinomen ist nach langer Persistenz der Papillome möglich.

3. Patientinnen (Alter 6, 39, 53 Jahre) wiesen perianale Papilloma-Warzen (verrucae) auf und wurden mit einer Zubereitung folgender Zusammensetzung behandelt:

| | |
|---|---|
| Methylparaben PH Eur | 0,61 g |
| Polyparaben PH Eur | 0,33 g |
| Kaliumsorbat PH Eur | 1,27 g |
| gereinigtes Wasser PH Eur | 993,48 g |
| Zitronenaroma | 1,00 g |
| Pfefferminzöl | 0,20 g |
| Zitronensäure | 2,00 g |
| Natriumselenit Pentahydrat | 1,11 g |
| Aerosil 200 | 30,0 g |
| | 1.030,00 g |

Die silicathältige Selenitlösung wurde in der Dosierung 5 x täglich 3 - 5 Tropfen auf die betroffenen Hautstellen aufgetragen. Bei allen 3 Patientinnen verschwanden die Papilloma-Warzen innerhalb eines Zeitraumes von 3 Wochen. Nach Verschwinden der Warzen wurde die Behandlung beendet.

## Patentansprüche

1. Verwendung von selenithältigen Verbindungen und pharmazeutisch akzeptablen Säuren, ausgewählt aus Zitronensäure, Essigsäure, Apfelsäure, Kohlensäure, Schwefelsäure, Salpetersäure, Salzsäure, diverse Fruchtsäuren oder Mischungen davon, zur Herstellung eines Mittels zur topischen oder bukkalen Anwendung bzw. zur mukosalen Verabreichung bei der Behandlung von Infektionen mit Papillomaviren.

2. Verwendung nach Anspruch 1, **dadurch gekennzeichnet, dass** das Mittel in wässeriger Lösung vorliegt und einen pH von unter 7,0, vorzugsweise unter 5,0, insbesondere von 3,0 bis 2,5, aufweist.

3. Verwendung nach einem der Ansprüche 1 oder 2, **dadurch gekennzeichnet, dass** die Säuren ausgewählt sind aus Zitronensäure, Essigsäure, Apfelsäure, Kohlensäure, diversen Fruchtsäuren oder Mischungen davon.

4. Verwendung nach einem der Ansprüche 1 bis 3, **dadurch gekennzeichnet, dass** das Mittel als Salbe vorliegt.

5. Verwendung nach einem der Ansprüche 1 bis 4, **dadurch gekennzeichnet, dass** das Mittel als Gel oder Emulsion vorliegt.

6. Verwendung nach einem der Ansprüche 1 bis 5, **dadurch gekennzeichnet, dass** das Mittel einen pharmazeutisch akzeptablen Träger, insbesondere Silikate, umfasst.

7. Verwendung nach einem der Ansprüche 1 bis 6, **dadurch gekennzeichnet, dass** das Mittel auf Trägermaterialien, vorzugsweise medizinische Schwämme und/oder andere adsorbierende Materialien, insbesondere Wundkegel, aufgetragen ist.

## Claims

1. A use of selenite-containing compounds and pharmaceutically acceptable acids selected from citric acid, acetic acid, malic acid, carbonic acid, sulfuric acid, nitric acid, hydrochloric acid, various fruit acids or mixtures thereof, for the preparation of an agent intended for topical or buccal application or mucosal administration, in the treatment of infections with papillomaviruses.

2. The use according to claim 1, **characterized in that** said agent is present in an aqueous solution and has a pH of below 7.0, preferably below 5.0 and, in particular, from 3.0 to 2.5.

3. The use according to any one of claims 1 or 2, **characterized in that** said acids are selected from citric acid, acetic acid, malic acid, carbonic acid, various fruit acids or mixtures thereof.

4. The use according to any one of claims 1 to 3, **characterized in that** said agent is present as an ointment.

5. The use according to any one of claims 1 to 4, **characterized in that** said agent is present as a gel or emulsion.

6. The use according to any one of claims 1 to 5, **characterized in that** said agent comprises a pharmaceutically acceptable carrier, in particular, silicates.

7. The use according to any one of claims 1 to 6, **characterized in that** said agent is applied onto carrier materials, preferably medical sponges and/or other adsorbing materials, in particular, wound cones.

## Revendications

1. Utilisation de composés contenant du sélénite et d'acides pharmaceutiquement acceptables, choisis parmi l'acide citrique, l'acide acétique, l'acide malique, l'acide carbonique, l'acide sulfurique, l'acide nitrique, l'acide chlorhydrique, divers acides de fruits ou des mélanges de ceux-ci, pour la préparation d'une composition destinée à l'application topique ou buccale ou pour l'administration sur les muqueuses dans le traitement d'infections par des papillomavirus.

2. Utilisation selon la revendication 1, **caractérisée en ce que** la composition se trouve en solution aqueuse et présente un pH inférieur à 7,0, de préférence inférieur à 5,0, en particulier de 3,0 à 2,5.

3. Utilisation selon la revendication 1 ou 2, **caractérisée en ce que** les acides sont choisis parmi l'acide citrique, l'acide acétique, l'acide malique, l'acide carbonique, divers acides de fruits ou des mélanges de ceux-ci.

4. Utilisation selon l'une quelconque des revendications 1 à 3, **caractérisée en ce que** la composition se trouve sous forme de pommade.

5. Utilisation selon l'une quelconque des revendications 1 à 4, **caractérisée en ce que** la composition se trouve sous forme de gel ou d'émulsion.

6. Utilisation selon l'une quelconque des revendications 1 à 5, **caractérisée en ce que** la composition comprend un véhicule pharmaceutiquement acceptable, en particulier des silicates.

7. Utilisation selon l'une quelconque des revendications 1 à 6, **caractérisée en ce que** la composition est appliquée sur des matériaux de support, de préférence des éponges médicales et/ou d'autres matériaux adsorbants, en particulier des cônes adsorbants.
